# EUROPÄISCHE PATENTSCHRIFT

(11) **EP 2 740 799 B1**
(45) Veröffentlichungstag und Bekanntmachung des Hinweises auf die Patenterteilung: **03.06.2020**
(21) Anmeldenummer: 13005644.3
(22) Anmeldetag: 04.12.2013
(51) Int. Cl.: C12P 3/00, C12P 5/02, C12P 7/06, C12P 7/16, C12P 7/52, C12P 7/54, C12P 7/56, C12P 7/64, C12M 1/00, C12M 1/04, C12M 1/107, C12M 1/34

(54) **Verfahren zur Herstellung von Kraftstoffen unter Verwendung von Algen und Pansen-Mikroorganismen**
Procecss for producing fuel employing algae and ruminal microorganisms
Procédé de production de carburants en utilisant des algues et des micro-organismes ruminaux

(30) Priorität: 07.12.2012 DE 102012023907; 12.02.2013 DE 102013002332
(43) Veröffentlichungstag der Anmeldung: 11.06.2014
(73) Patentinhaber: Airbus Defence and Space GmbH, 85521 Ottobrunn (DE)
(72) Erfinder: Reidt, Ulrich, 34613 Schwalmstadt (DE); Heller, Christoph, 82024 Taufkirchen (DE); Friedberger, Alois, 85667 Oberpframmern (DE); Müller-Wiesner, Detlef, 75009 Paris (FR); Petillon, Odile, 83250 Marquartstein (DE)
(74) Vertreter: LKGLOBAL Lorenz & Kopf PartG mbB Patentanwälte

(56) Entgegenhaltungen:
- EP-A1- 2 302 019
- EP-A2- 1 980 620
- WO-A2-2011/006019
- WO-A2-2011/047372
- WO-A2-2012/100093
- US-B1- 8 017 366

## Beschreibung

### BEREICH DER ERFINDUNG

Die vorliegende Erfindung betrifft ein Verfahren zur Gewinnung verwertbarer Produkte aus Biomasse, die aus der Produktion von Biokraftstoffen aus Algen hervorgeht. Die Verwertung der Biomasse erfolgt in einem Reaktor, welcher Mikroorganismen enthält.

### HINTERGRUND DER ERFINDUNG

In der Luftfahrt ist die Kerosinerzeugung aus Algen- oder Archaeenöl ein viel versprechender Prozess, um CO₂-neutrale Treib- und Kraftstoffe herzustellen. Bei der einstufigen biotechnologischen Produktion von Ölen aus Algen, Archaeen oder anderen Mikroorganismen fallen große Mengen an Biomasse an. Meistens wird diese Biomasse als Dünger oder als Viehfutter verwendet und nicht in weiteren biotechnologischen Schritten prozessiert und verstoffwechselt. Diese Biomasse stellt ein hohes energetisches Potenzial dar, was bisher weitestgehend ungenutzt blieb.

Die biotechnologische Vergasung von Biomasse zu Methan (Biogasanlage) ist ein bekannter Prozess, der schon mehrfach in der Literatur beschrieben worden ist. Auch das mikrobiologische Milieu im Rinderpansen ist gut untersucht und fast alle Mikroorganismen sind mit deren Stoffwechselwegen bekannt. Des Weiteren sind die Clostridien ein sehr gut erforschter Bakterienstamm, deren einzelne Arten schon heute für biotechnologische Prozesse genutzt werden.

Die US 2008/0187975 A1 beschreibt ein Verfahren zur anaeroben Digestion pflanzlicher Biomasse unter Verwendung im Pansen von Wiederkäuern vorkommender Mikroorganismen, um Wasserstoff, Methan oder Ethanol zu gewinnen. Es wird jedoch lediglich die Verwertung von pflanzlicher Biomasse, Siedlungsabfällen und Viehdung offenbart.

In der WO 2010/056304 A1 wird ein Verfahren zur Herstellung kurzkettiger Alkylalkohole offenbart, die aus pflanzlicher Biomasse mit Hilfe natürlicher, anaerober Mikroorganismen gewonnen werden. Eine Zielsetzung der WO 2010/056304 A1 ist die Herstellung einzelner Alkohole, insbesondere Ethanol, durch bestimmte Mikroorganismen, deren Stoffwechselprodukt ausschließlich den jeweiligen Alkohol umfasst. Hierzu eignen sich unter anderem auch Bakterien der Gattung Ruminococcus, welche aus dem Pansen von Wiederkäuern gewonnen werden können. Auch dieses Dokument beschreibt ein Verfahren, in dem ausschließlich pflanzliche Biomasse verwertet wird.

Bei den oben genannten Verfahren handelt es sich um einstufige Prozesse, bei denen die vorhergehende Produktion der Biomasse nicht in direkter Verknüpfung mit deren Verstoffwechslung zu verwertbaren Produkten steht.

Bei der Produktion von Ölen aus Algen und Archaeen handelt es sich bisher ebenfalls um einen einstufigen Prozess, wobei über Photosynthese aus CO₂ und Sonnenlicht Öl und Biomasse erzeugt werden (phototrophes Verfahren).

Die WO 2011/006019 A2 beschreibt Verfahren zum Verarbeiten einer Biomasse, zum Beispiel zur Herstellung von Lipid- und Nichtlipidmaterialien, indem die Autolyse einer Biomasse induziert wird. Die Biomasse kann unter Verwendung einer beliebigen Art von Mikroorganismen und eines beliebigen verfügbaren biologisch abbaubaren Substrats einschließlich Abfallmaterialien erzeugt werden. Die Lipid- und Nichtlipidmaterialien befinden sich in zwei separaten Schichten des Autolysats und können zur Herstellung von Produkten wie Biokraftstoffe und Nahrungsergänzungsmittel verwendet werden.

Die EP1980620 A2 richtet sich auf ein Verfahren zur Produktion von Biogas und Aminosäuren aus einem Hydrolysat und beschreibt, dass die Verarbeitung eines erneuerbaren Rohmaterials das Unterziehen des Materials einer enzymatischen Hydrolyse, das Trennen des Hydrolyseprodukts in feste und wässrige flüssige Fraktionen, die Verwendung der flüssigen Fraktion als Kohlenstoffquelle für einen Mikroorganismus, um eine Zielsubstanz durch Fermentation herzustellen, und das Verwenden der festen Fraktion, um Biogas durch anaerobe Vergärung herzustellen, umfasst.

Die EP2302019 A1 beschreibt ein System zur Kultivierung von Algen, Extraktion von Lipiden und Umesterung der Lipide, um Biodiesel zu erhalten. Das System besteht aus drei Bereichen, nämlich Kultivierung, Extraktion und Lagerung und Reaktion. Im Bereich der Lipidextraktion befindet sich ein Ultraschallreaktor, in dem die äußeren Wände der Algen zusammen mit denen des Ölsacks aufgerissen werden, um die Extraktion von Lipiden zu ermöglichen. Im Umesterungsbereich befindet sich ebenfalls ein Ultraschallreaktor, um die Reaktion zu beschleunigen. Die US 8,017,366 B1 beschreibt ein System zur Herstellung von Biokraftstoffen, die Methan, Ethanol und Biodiesel umfassen, umfassend einen Tank mit einem verschließbaren Deckel.

Die WO 2012/100093 A2 beschreibt ein Verfahren zur Herstellung von Biokraftstoffen, umfassend die Schritte
a. in einer ersten Stufe die Produktion von Biokraftstoffen aus Organismen ausgewählt aus Makroalgen und Mikroalgen unter Bildung von Biomasse, und
b. in einer zweiten Stufe die Umsetzung der Biomasse aus der ersten Stufe zu niedermolekularen Verbindungen in einem Reaktor, welcher im Pansen von Wiederkäuern vorkommende Mikroorganismen enthält,
dadurch gekennzeichnet, dass es sich bei der ersten Stufe um einen aeroben Prozess und bei der zweiten Stufe um einen anaeroben Prozess handelt und dass die erste aerobe Stufe von der zweiten anaeroben Stufe durch eine Vorrichtung getrennt ist, welche eine Zuleitung der in der zweiten Stufe entstehenden Metabolite in die erste Stufe ermöglicht.

Sie beschreibt weiterhin ein System zur Herstellung von Biokraftstoffen, aufweisend:
a. einen Reaktor (i) zur Produktion von Biokraftstoffen aus Organismen ausgewählt aus Makroalgen und Mikroalgen in einer ersten Stufe in einem aeroben Bereich unter Bildung von Biomasse und
b. einen Reaktor (ii) zur Umsetzung der Biomasse aus dem Reaktor (i) zu niedermolekularen Verbindungen in einer zweiten Stufe in einem anaeroben Bereich, welcher im Pansen von Wiederkäuern vorkommende Mikroorganismen enthält, wobei
   a. der Reaktor (i) einen Einlass zum Zuführen der Organismen, einen Auslass zur Abgabe der erzeugten Biokraftstoffe, einen Auslass zur Abgabe der erzeugten Biomasse an den Reaktor (ii) und einen Einlass zur Rückführung der im Reaktor (ii) erzeugten Metabolite aufweist und
   b. der Reaktor (ii) einen Einlass zum Zuführen der Biomasse aus dem Reaktor (i), einen Auslass zur Rückführung der erzeugten Metabolite in den Reaktor (i) und einen Auslass für die erzeugten niedermolekularen Verbindungen aufweist.

### ZUSAMMENFASSUNG DER ERFINDUNG

Es besteht daher ein Bedarf an einer umfassenderen Verwertung von Biomasse, um die aus der Algenölproduktion hervorgehende Biomasse weiter umzusetzen, beispielsweise in verwertbare Produkte. Ein solches Verfahren besäße eine wesentlich höhere Effizienz in Bezug auf Energieausbeute und Energiebilanz. Daher ist es eine Aufgabenstellung der vorliegenden Erfindung, ein mehrstufiges Verfahren bereitzustellen, worin dem einstufigen Prozess der Algenölproduktion ein zweiter, lichtunabhängiger Prozess (chemolithotrophes Verfahren) angeschlossen wird. Hierbei kann die aus der Algenölproduktion hervorgehende Biomasse in einen Bioreaktor überführt und dort zu verwertbaren Produkten verstoffwechselt werden.

Der hierzu verwendete Bioreaktor orientiert sich an den Stoffwechselvorgängen im Rinderpansen und bildet diese nach (artifizieller Pansen). Die Verwendung von Mikroorganismen aus dem Pansen zur Verstoffwechslung von Biomasse stellt eine äußerst energieeffiziente Methode dar.

Gemäß einem ersten Teilaspekt wird ein Verfahren zur Herstellung von Biokraftstoffen bereitgestellt, umfassend die Schritte
a. in einer ersten Stufe die Produktion von Biokraftstoffen aus Organismen ausgewählt aus Makroalgen, Mikroalgen, ethanolproduzierenden Algen und/oder Archaeen unter Bildung von Biomasse, und
b. in einer zweiten Stufe die Umsetzung der Biomasse aus der ersten Stufe zu niedermolekularen Verbindungen in einem Reaktor, welcher im Pansen von Wiederkäuern vorkommende Mikroorganismen enthält (artifizieller Pansen),
dadurch gekennzeichnet, dass es sich bei der ersten Stufe um einen aeroben Prozess und bei der zweiten Stufe um einen anaeroben Prozess handelt und dass die erste aerobe Stufe von der zweiten anaeroben Stufe durch eine Vorrichtung getrennt ist, welche eine Diffusion der in der zweiten Stufe entstehenden Metabolite in die erste Stufe ermöglicht.

Gemäß der vorliegenden Erfindung ist daher in einer ersten Stufe die Produktion von Biokraftstoffen aus Organismen ausgewählt aus Makroalgen, Mikroalgen, ethanolproduzierenden Algen und/oder Archaeen unter Bildung von Biomasse vorgesehen. In einer zweiten Stufe ist die Umsetzung der Biomasse aus der ersten Stufe zu niedermolekularen Verbindungen in einem Reaktor vorgesehen, welcher im Pansen von Wiederkäuern (auch als Rumen bezeichnet) vorkommende Mikroorganismen enthält (artifizieller Pansen). Somit besitzt das Verfahren der vorliegenden Erfindung den Vorteil, das energetische Potential der eingesetzten Rohstoffe besser nutzen zu können. Insbesondere kann die in der ersten Stufe erzeugte Biomasse gewinnbringend für die Erzeugung von niedermolekularen Verbindungen genutzt werden.

Gemäß einer bevorzugten Ausführungsform werden in der ersten Stufe als Biokraftstoffe Biodiesel, Bioethanol, Biogas und Biowasserstoff hergestellt (phototrophes Verfahren).

Der Begriff "Biokraftstoff" bezieht dabei sich auf einen flüssigen oder gasförmigen Kraftstoff, welcher aus Biomasse hergestellt wird. Demzufolge handelt es sich bei "Biodiesel", "Bioethanol", "Biogas" und "Biowasserstoff" um die aus der Biokraftstoff-Produktion hervorgehenden Kraftstoffe Diesel, Ethanol, Gas (z.B. Methan) und Wasserstoff.

Gemäß einer bevorzugten Ausführungsform handelt es sich bei den in der zweiten Stufe hergestellten niedermolekularen Verbindungen um Methan, Wasserstoff, Butanol, Ethanol, Essigsäure, Aceton, Buttersäure und/oder Milchsäure.

Bei den in der ersten Stufe verwendeten Organismen handelt es sich um Makroalgen, Mikroalgen, ethanol-produzierende Algen und Archaeen, bevorzugt um mindestens eine der Gattungen und Arten ausgewählt aus der Gruppe umfassend Braunalgen, Laminaria ssp., Palmaria ssp.,Chlorella ssp., Spirulina ssp., Sargassum ssp., Gracilaria ssp., Dunaliella ssp., Prymnesium parvum, Euglena ssp. und Archaeen.

Gemäß einer besonders bevorzugten Ausführungsform kann die erste Stufe eine oder mehrere der folgenden Organismen umfassen:
Cutleria Greville, Microzonia velutina, Desmarestia aculeata, Padina pavonica, Ectocarpales Bessey, Ascophyllum nodosum, Fucus serratus, Fucus spiralis, Fucus vesiculosus, Macrocystis pyrifera, Saccharina latissima, Saccharina japonica, Spirulina platensis, Spirulina fusiformis, Spirulina maxima, Palmaria palmate, Sargassum acinarium, Sargassum bermudense, Sargassum dentifolium, Sargassum desfontainesii, Sargassum elegans, Sargassum fissifolium, Sargassum fluitans, Sargassum grevillei, Sargassum ilicifolium, Sargassum incisifolium, Sargassum muticum, Sargassum natans, Sargassum oligocystum, Sargassum polyceratium, Sargassum tenerimum, Sargassum vulgare, Gracilaria bursa-pastoris, Gracilaria multipartite, Gracilaria gracilis, Gracilariopsis, Gracilaria verrucosa, Gracilaria confervoides, Methanococcus sp., Methanococcus jannasehii, Methanococcus maripaludis, Methanothermus sp., Methanothermus fervidus, Methanobacterium sp., Methanobacterium formicicum, Methanococcus voltae, Methanobacterium thermoautotrophicum, Methanoculleus bourgense, Methanofollis tationis, Methanospirillum hungatei, Methanosarcina mazei, Methanosaeta concilii, Methanothermobacter thermoautotrophicus, Methanocaldococcus jannasehii, Methanocella sp., Methanocella paludicola, Methanocella arvoryzae, Methanosarcina barkeri, Laminaria abyssalis, Laminaria agardhii, Laminaria appressirhiza, Laminaria brasiliensis, Laminaria bullata, Laminaria complanata, Laminaria cordata, Laminaria ephemera, Laminaria farlowii, Laminaria hyperborean, Laminaria inclinatorhiza, Laminaria longipes, Laminaria nigripes, Laminaria ochroleuca, Laminaria pallida, Laminaria platymeris, Laminaria rodriguezii, Laminaria ruprechtii, Laminaria setchellii, Laminaria sinelairii, Laminaria solidungula, Laminaria yezoensis, L. angustata, L. eichorioides, L. eoricea, L. dentigera, L. diabolica, L. groenlandica, L. japonica, L. Kombu, L. longicruris, L. longipedalis, L. longissima, L. ochotensis, L. religiosa, L. saccharina, Chlorella angustoellipsoidea, Chlorella botryoides, Chlorella capsulate, Chlorella emersonii, Chlorella fusea, Chlorella homosphaera, Chlorella luteo-viridis, Chlorella marina, Chlorella miniata, Chlorella minutissima, Chlorella mirabilis, Chlorella ovalis, Chlorella parasitica, Chlorella peruviana, Chlorella rugosa, Chlorella saccharophila, Chlorella salina, Chlorella spaerckii, Chlorella sphaerica, Chlorella stigmatophora, Chlorella subsphaerica, Chlorella trebouxioides, Chlorella vulgaris, Dunaliella salina, Dunaliella tertiolecta, D. acidophila, D. bardawil, D. bioculata, D. lateralis, D. maritime, D. minuta, D. parva, D. peircei, D. polymorpha, D. primolecta, D. pseudosalina, D. quartolecta, Prymnesium parvum, Euglena gracilis, Euglena carterae, Euglena deses, Euglena ehrenbergii, Euglena haematoda, Euglena intermedia, Euglena longa, Euglena piseiformis, Euglena quartana, Euglena sanguinea, Euglena terricola, Euglena texta, Euglena variabilis, Euglena viridis, Lepocinclis acus, Lepocinclis oxyuris, Lepocinclis platydesma, Lepocinclis spirogyroides, und Lepocinclis tripteris.

Gemäß einer bevorzugten Ausführungsform kann die zweite Stufe (artifizieller Pansen) mindestens einen der Mikroorganismen ausgewählt aus der Gruppe umfassend Ruminococcus ssp., Ralstonia ssp., Lactobacillus ssp., Clostridium ssp., Bacterioides ssp., Archaeen, Isotrichidae, Ophryoscolecidae, Ciliata, Hefen und Pilze umfassen, wobei bevorzugt mindestens eine der Bakterienarten ausgewählt aus der Gruppe Clostridium acetobutylicum, Clostridium beijerinckii, Ruminococcus flavefaciens, Ruminococcus albus, Ralstonia eutropha und Hydrogenomonas eutrophus anwesend sein soll.

Gemäß einer besonders bevorzugten Ausführungsform kann die zweite Stufe mindestens eine der folgenden Organismen umfassen:
Clostridium acetobutylicum, Clostridium beijerinckii, Clostridium haemolyticum, Clostridium novyi, Clostridium oedematiens, Clostridium coccoides, Ruminococcus albus, Ruminococcus bromii, Ruminococcus callidus, Ruminococcus flavefaciens, Ruminococcus hansenii, Ruminococcus hydrogenotrophicus, Ruminococcus lactaris, Ruminococcus luti, Ruminococcus obeum, Ruminococcus pro ductus, Ruminococcus schinkii, Ruminococcus torques, Bacteroides cellulosolvens, Butyricicoccus pullicaecorum, Gemmiger formicilis, Oscillibacter valericigenes, Saccharofermentans acetigenes, Anaerosporobacter mobilis, Desulfotomaculum guttoideum, Fusobacterium naviforme, Lactobacillus rogosae, Lactonifactor longoviformis, Bacteroides ruminicola, Streptococcus bovis, Selenomonas lactilytica, Methanobrevibacter ruminantium, Blautia wexlerae, Blautia coccoides, Blautia hansenii, Blautia hydrogenotrophica, Blautia luti, Entodinium caudatum, Isotricha prostoma, Fibrobacter succinogenes, Neocallimastix frontalis, Neocallimastix patriciarum, Orpinomyces joyonii, Orpinomyces sp., Piromyces equi, Piromyces sp., Piromyces rhizinata, Prevotella ruminicola, Neocallimastix patriciarum, Piromyces rhizinfata, Butyrivibrio fibrisolvens, Prevotella albensis, Eubacterium ruminantium, , Piromyces communis, Prevotella bryantii, Pseudobutyrivibrio xylanivorans,.

Bei den gemäß der vorliegenden Erfindung verwendeten Algen, Archaeen, Mikroorganismen und Organismen kann es sich um natürlich vorkommende Arten handeln, oder auch um gezüchtete oder gentechnisch veränderte Arten.

Viele sehr ertragreiche Algenarten wachsen ausschließlich mixotroph. Diese Algen benötigen neben Licht und CO₂ zusätzlich eine Kohlenstoffquelle. Eine potentielle Kohlenstoffquelle kann die aus der zweiten Stufe prozessierte Biomasse sein. Diese kann in Form eines Kreislaufes der ersten Stufe wieder zugeführt werden. Bevorzugt kann bei diesem Kreislauf ein Teil der in der zweiten Stufe anfallenden Produkte (Metabolite) von den Organismen der ersten Stufe verstoffwechselt werden. So ist eine Verstoffwechslung von Zellulose oder Xylose in Ein- und/oder Zweifachzucker in der zweiten Stufe möglich, die der ersten Stufe wieder zugeführt werden können. Es kann daher ein Kreislauf entstehen, wobei die Biomasse der ersten Stufe (aus Algen oder Archaeen) in der zweiten Stufe durch Organismen in einem Reaktor (artifizieller Pansen) umgesetzt wird, und die in der zweiten Stufe durch die Organismen im artifiziellen Pansen entstehenden Produkte, wie beispielsweise Ein- oder Zweifachzucker, wieder der ersten Stufe zugeführt werden, um dort bei der Produktion von Biomasse und Ölen Verwendung zu finden.

Eine solche Vereinigung der ersten Stufe mit der zweiten Stufe zu einem einzigen symbiotischen System (Anreicherungskultur) kann den Ertrag an wünschenswerten Produkten erheblich steigern. Dieses System beinhaltet einen aeroben phototrophen Bereich (erste Stufe) und einen anaeroben Bereich (zweite Stufe), wobei die im anaeroben Bereich entstehenden Metabolite dem aeroben Bereich zugeführt werden. In einer besonders bevorzugten Ausführungsform können dabei beide Stufen durch eine luftundurchlässige Schicht (semipermeable Membran) voneinander getrennt sein, welche für die im anaeroben Bereich entstehenden Metabolite durchlässig ist.

Gemäß einer anderen Ausführungsform kann die erste Stufe sowohl im offenen als auch im geschlossenen System durchgeführt werden.

Der Begriff "offenes System" bezieht sich dabei auf einen Reaktor, welcher einen permanenten Energie- und Materieaustausch mit der Umgebung ermöglicht. Das System ist nicht abgeschlossen. Hierzu eignet sich beispielsweise das "open Pond"-Verfahren.

Im Gegensatz dazu stellt ein "geschlossenes System" einen Reaktor dar, welcher gegebenenfalls Energie, beispielsweise durch Wärme- oder Strahlungszufuhr, jedoch keine Materie mit der Umgebung austauschen kann. Somit kann eine Kontamination des Reaktionsmediums durch Stoffe und Organismen aus der Umgebung verhindert werden.

Gemäß der Erfindung handelt es sich bei der Algenölproduktion (erste Stufe) um einen aeroben Prozess und bei der Umsetzung der Biomasse im artifiziellen Pansen um einen anaeroben Prozess, aus dem bevorzugt mindestens einer oder mehrere der folgenden Metabolite hervorgeht: Kohlenwasserstoffe, organische Säuren, Mineralien, Kaliverbindungen, Phosphatverbindungen, Stickstoffverbindungen, Ein- und/oder Zweifachzucker und Proteine. Die in der zweiten Stufe entstehenden Metabolite werden der ersten Stufe direkt zugeführt. Besonders bevorzugt sind beide Stufen durch eine luftundurchlässige Schicht (semipermeable Membran) voneinander getrennt, welche für die im anaeroben Bereich entstehenden Metabolite durchlässig ist, wobei es sich weiter bevorzugt um eine Lehm- oder Erdschicht handelt.

Gemäß einer besonders bevorzugten Ausführungsform wird mindestens einer oder mehrere der folgenden Metabolite in der zweiten Stufe erzeugt und in der ersten Stufe für ein Wachstum der Organismen eingesetzt:
Kohlenwasserstoffe, CO₂, NH₃, Harnsäure, organische Säuren, Stärke, Dextrin, Pyruvat, Glykogen, Acetat, Butyrat, Lactat, Formiat, Mineralien, Kaliverbindungen, metallische Verbindungen (Fe, Cu, Mg, Mn, Li), Vitamine, Biotin, Glycerin, ATP, FAD, NAD, NADP, Thiamin, Riboflavine, Liponsäuren, Alkohole, Phosphatverbindungen, Schwefelverbindungen, Stickstoffverbindungen, Zuckerverbindungen,

Proteine, Chinone, Coenzyme und prosthetische Gruppen, Nukleotide, Ein- und/oder Zweifachzucker.

Gemäß einer bevorzugten Ausführungsform kann in einem mixotrophen Verfahren neben den oben genannten Organismen auch mindestens einer der folgenden Organismen in der ersten oder zweiten Stufe anwesend sein:
Chlamydomonas globosa, Chlamydomonas moewusii, Chlamydomonas nivalis, Chlamydomonas reinhardtii, Chlorella minutissima, Scenedesmus bijuga, Actinastrum aciculare, Actinastrum bacillare, Actinastrum fluviatile, Actinastrum gracillimum, Actinastrum guttula, Actinastrum hantzschii, Actinastrum rhaphidioides, Coelastrum astroideum, Coelastrum cambricum, Coelastrum chodatii, Coelastrum crenatum, Coelastrum giganteum, Coelastrum indicum, Coelastrum irregulare, Coelastrum microporum, Coelastrum morus, Coelastrum naegelii, Coelastrum printzii, Coelastrum probiscideum, Coelastrum pseudomicroporum, Coelastrum pulchellum, Coelastrum pulchrum, Coelastrum reticulatum, Coelastrum schizodermaticum, Coelastrum sphaericum, Coelastrum stuhlmanii, Coelastrum triangulare, Coelastrum verrucosum, Crucigenia australis, Crucigenia cordata, Crucigenia fenestrata, Crucigenia floralis, Crucigenia lauterbomii, Crucigenia quadrata, Crucigenia tetrapedia, Crucigenia triangularis, Crucigenia truncate, Dicellula geminate, Scenedesmus obtusus, Tetradesmus wisconsinensis, Tetrallantos lagerheimii, Tetrallantos novae-geronae, Tetrastrum asymmetricum, Tetrastrum constrictum, Tetrastrum elegans, Tetrastrum glabrum, Tetrastrum heteracanthum, Tetrastrum komarekii, Tetrastrum parallelum, Tetrastrum staurogeniaeforme, Tetrastrum tenuispinum, Tetrastrum triacanthum, und Tetrastrum triangulare.

Ein zweiter Teilaspekt der vorliegenden Erfindung betrifft die Verwendung eines Reaktors als artifizieller Pansen. Ein derartiger Reaktor verfügt über Vorrichtungen zur Zufuhr von Biomasse (Zufütterungsstelle) sowie zur Entnahme der niedermolekularen Verbindungen. Eine Vorrichtung zur Zufuhr von Reagenzien ist gegebenenfalls von Vorteil, um beispielsweise die Zugabe von Säure oder Lauge zur Einstellung des pH-Wertes zu ermöglichen. Außerdem verfügt der Reaktor über eine Begasungsanlage zur Herstellung einer anaeroben Atmosphäre.

Gemäß einer bevorzugten Ausführungsform kann der Reaktor im Chargenprozess ("Batch-Prozess") betrieben werden, wobei dem Reaktor eine auf dessen Fassungsvermögen begrenzte Menge an Biomasse zugeführt wird und nach beendeter Umsetzung die Produkte entnommen werden. Alternativ dazu kann der Reaktor auch im Zulauf-Verfahren ("Fed-Batch-Prozess") betrieben werden. Hierbei werden dem Reaktor kontinuierlich Biomasse zugeführt und Produkte entnommen.

In einer bevorzugten Ausführungsform ist der Zufütterungsstelle eine Vorrichtung zur Zerkleinerung von Biomasse vorgeschaltet. Die automatische Zufuhr zum artifiziellen Pansen erfolgt bevorzugt durch eine Transportschnecke.

Zur Messung der Bedingungen im Reaktor und um die Erkennung einer möglichen Kontamination frühzeitig zu ermöglichen, verfügt der Reaktor in einer bevorzugten Ausführungsform über eine Quasi-Echtzeit-Biodetektion, welche Bakterien und andere Mikroorganismen detektieren kann. Mögliche Komponenten dieser Quasi-Echtzeit-Biodetektion werden im folgenden näher beschrieben.

In der ersten Stufe (phototrophes Verfahren) kann Sensorik eingesetzt werden, welche die Lichtverhältnisse erfasst. Neben der Lichtintensität kann auch das Lichtspektrum erfasst werden, um die Prozesse optimieren zu können. Außerdem kann die Messung des Chlorophyll- und des Carotinoidgehalts der phototrophen Organismen mittels Absorptionsmethoden oder Fluoreszenzmethoden erfolgen, da diese Werte Aufschluss über den physiologischen Zustand der phototrophen Organismen geben können.

Die Produkte aus der ersten und zweiten Stufe können jeweils analysiert werden. Insbesondere lässt sich mit einer Biodetektion die mikrobielle Qualität erfassen, wenn die Produkte der Nahrungs- und Futtermittelkette zugeführt werden sollen. Der Begriff "mikrobielle Qualität" bezieht sich dabei auf die Reinheit der Produkte in Bezug auf eine mögliche Kontamination mit Fremdorganismen.

Sowohl der Photobioreaktor (erste Stufe) als auch der Reaktor des artifiziellen Pansen (zweite Stufe) können über mindestens einen oder mehrere der folgenden Gas-Sensoren gesteuert und überwacht werden:
- CO₂-Sensor
- Kohlenwasserstoffsensor
- O₂-Sensor
- H₂-Sensor
- Elektronische Nase

Daneben können die folgenden indirekten Parameter über eine entsprechende Sensorik permanent in beiden Stufen erfasst werden:
- pH-Wert über eine pH-Sensorik
- Trübung über eine Messung der optischen Dichte bei 600 nm in einem Photometer zur Aufnahme einer Wachstumskurve
- Schaumbildung (Foaming) über einen Kontaktsensor

Der Begriff "Wachstumskurve" bezieht sich dabei auf die Anzahl der anwesenden Organismen in Abhängigkeit von der Zeit.

In einer bevorzugten Ausführungsform verfügen alle angeschlossenen Sensoren über einen Rückkopplungsmechanismus, d.h., einen Regelungsmechanismus, so dass bei einem Überschreiten eines bestimmten Parameters umgehend Gegenmaßnahmen erfolgen können. Am Beispiel der pH-Sensorik kann bei einem Überschreiten bzw. Unterschreiten eines bestimmten pH-Wertes die Reaktionsmischung durch eine Säure oder eine Lauge gegengepuffert werden.

Nach Aufschluss einer bestimmten Menge Organismen können der DNA-Gehalt durch Absorption bei 260 nm Wellenlänge und der Guanin- und Cytosingehalt (GC-Gehalt) bestimmt werden. Der GC-Gehalt im Genom kann als taxonomisches Merkmal zur Einteilung der Organismen, insbesondere der Bakterien verwendet werden, um zur Auswertung des Verfahrens deren Gattung und/oder Art zu bestimmen.

Insbesondere kann das Verfahren der Durchflusszytometrie für die Erfassung der Zellkonzentration zur Anwendung kommen. Bei diesem Verfahren fließen fluoreszenzmarkierte Zellen mit hoher Geschwindigkeit in einer Kapillare an einer elektrischen Spannung oder einem Lichtstrahl vorbei und werden über einen Detektor einzeln erfasst. Das Verfahren kann sowohl spezifisch als auch unspezifisch angewendet werden. Bei der spezifischen Detektion werden die Zellen zuvor mit Hilfe von fluoreszenzmarkierten Antikörpern markiert, während bei der unspezifischen Detektion häufig interkalierende Fluoreszensfarbstoffe eingesetzt werden. Auch über eine Vitalfärbung lässt sich der physiologische Zustand der Zellen sehr gut erfassen. Der Gehalt an Chlorophyll oder Phycoerythrin lässt sich über die Fluoreszenz direkt erfassen.

Enzymatische Tests bzw. biochemische Tests können eine Aussage über die Vitalität der kraftstofferzeugenden Organismen geben. Auch klassische kinetische Assays können den Vitalitätszustand der Zellen wiedergeben.

Folgende Parameter können wahlweise neben Trübungsmessung und Chlorophyllgehalt zur Aufnahme einer Wachstumskurve herangezogen werden:
- Messung der ATP-Konzentration
- Messung des NADP bzw. NAD-Gehalts
- Messung des Proteingehalts bei 280 nm
- Messung der Nukleotidkonzentration bei 260 nm
- Trockenmassenbestimmung

In einer bevorzugten Ausführungsform ist dem Durchflusszytometer eine Vorrichtung zur automatischen Probenentnahme vorgeschaltet, welche bevorzugt mit einer integrierten vollautomatischen Fluoreszenzfärbung ausgestattet ist.

Die zur Identifizierung von Organismen zur Verfügung stehenden analytischen Verfahren, die nach einer Probenentnahme angewendet werden können, umfassen
- Bestimmung der koloniebildenden Einheiten (KBE) durch Kultivierung auf Nährböden bzw. Selektivnährböden. Insbesondere könnte bei diesem Verfahren die Membranfilter-Mikrokolonie-Fluoreszenzmethode zum Einsatz kommen.
- PCR
- Sequenzierung mit einem Markergen, insbesondere 16SrRNA-Gen oder OmpS-Gen
- ELISA (Antikörper)
- FISH
- Aptamerdetektion
- In situ-Hybridisierung
- Westernblot
- Northernblot
- Southernblot
- Mikroarray
- Raman-Spektroskopie
- Elektronische Nase, Aufnahme eines spezifischen Geruchsprofils
- Detektion über Phagenproteine (nur bei Bakterien)
- Spezifische Durchflusszytometrie

Ein weiterer Aspekt der vorliegenden Erfindung betrifft ein System zur Herstellung von Biokraftstoffen, aufweisend eine erste aerobe Stufe (1), welche die Produktion von Biokraftstoffen aus Makroalgen, Mikroalgen, ethanolproduzierenden Algen und/oder Archaeen unter Bildung von Biomasse umfasst, und eine zweite anaerobe Stufe (2), welche die Umsetzung der Biomasse aus der ersten Stufe zu niedermolekularen Verbindungen in einem Reaktor, welcher im Pansen von Wiederkäuern vorkommende Mikroorganismen enthält (artifizieller Pansen), umfasst, wobei die erste Stufe (1) einen Einlass (24) zum Zuführen der Algen, einen Auslass (25) zur Abgabe der erzeugten Biokraftstoffe, einen Auslass (3) zur Abgabe der erzeugten Biomasse an die zweite Stufe und einen Einlass (4) zur Rückführung der in der zweiten Stufe erzeugten Metabolite in Form einer Membran aufweist und die zweite Stufe (2) einen Einlass (3) zum Zuführen der Biomasse aus der ersten Stufe, einen Auslass (4) zur Rückführung der erzeugten Metabolite in die erste Stufe in Form einer Membran und einen Auslass (26) für die erzeugten niedermolekularen Verbindungen aufweist.

Diese und andere Aspekte der vorliegenden Erfindung werden nun mit Verweis auf die nachfolgend beschriebenen Ausführungsformen näher erläutert.

### KURZE BESCHREIBUNG DER FIGUREN

Nachfolgend wird anhand der beigefügten Zeichnungen näher auf Ausführungsformen der Erfindung eingegangen. Es zeigen:
- **Fig. 1**: eine schematische Darstellung eines zweistufigen Verfahrens
- **Fig. 2**: eine Ausführungsform der Erfindung (mixotrophes Verfahren)
- **Fig. 3**: eine Ausführungsform der Erfindung (gemischte symbiotisches Verfahren)
- **Fig. 4**: eine Darstellung eines artifiziellen Pansen
- **Fig. 5**: eine Darstellung eines dem artifiziellen Pansen vorgeschalteten Zerkleinerers
- **Fig. 6**: eine schematische Darstellung eines artifiziellen Pansen mit nachgeschalteter automatischer Probenentnahme und Durchflusszytometer
- **Fig. 7**: eine schematische Darstellung möglicher Ausführungsformen

### AUSFÜHRLICHE BESCHREIBUNG DER AUSFÜHRUNGSFORMEN

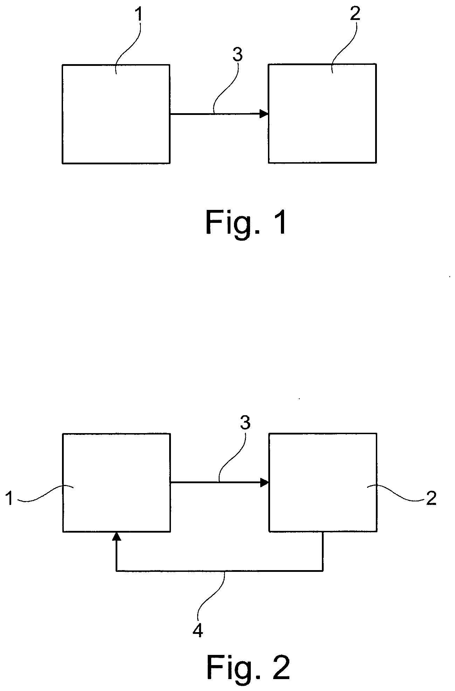

Fig. 1 zeigt ein Beispiel, worin in einer ersten Stufe 1 die Produktion von Biokraftstoffen aus Algen unter Bildung von Biomasse erfolgt. Bei der ersten Stufe handelt es sich um die Produktion von Algenöl, beispielsweise in einem phototrophen Verfahren. Bei den hergestellten Biokraftstoffen kann es sich um beispielsweise um Biodiesel, Bioethanol, Biogas und Biowasserstoff handeln. Es können in der ersten Stufe eine oder mehrere der oben genannten Algenarten anwesend sein. In einer zweiten Stufe 2 erfolgt die Umsetzung der Biomasse aus der ersten Stufe zu niedermolekularen Verbindungen, wobei es sich beispielsweise um Methan, Wasserstoff, Butanol, Ethanol, Essigsäure, Aceton, Buttersäure und/oder Milchsäure handelt. Für die zweite Stufe ist ein Reaktor vorgesehen, welcher im Pansen von Wiederkäuern vorkommende Mikroorganismen enthält (artifizieller Pansen). Dabei handelt es sich um eine oder mehrere der oben genannten Organismen. Die bei der Algenölproduktion entstandene Biomasse wird über eine Auslass- bzw. Zufuhrvorrichtung 3 dem artifiziellen Pansen zugeführt.

Eine Ausführungsform eines mixotrophen Verfahrens wird in Fig. 2 dargestellt. Hierbei können Metabolite, die neben den oben genannten Produkten aus der zweiten Stufe 2 hervorgehen, in Form eines Kreislaufs der ersten Stufe 1 wieder zugeführt werden und den dort anwesenden Algen als Nahrungsquelle dienen. Dabei handelt es sich bei der ersten Stufe um einen aeroben und bei der zweiten Stufe um einen anaeroben Prozess. Beide Stufen sind durch eine Einlass- bzw. Auslassvorrichtung 4 miteinander verbunden, welche die Zuführung der Metabolite von der zweiten in die erste Stufe ermöglicht. Im mixotrophen Verfahren können in der ersten und zweiten Stufe weitere Organismen anwesend sein, welche oben genannt sind. Aus der ersten Stufe geht bevorzugt mindestens einer der folgenden Metabolite hervor: Kohlenwasserstoffe, organische Säuren, Mineralien, Kaliverbindungen, Phosphatverbindungen, Stickstoffverbindungen, Ein- und/oder Zweifachzucker und Proteine.

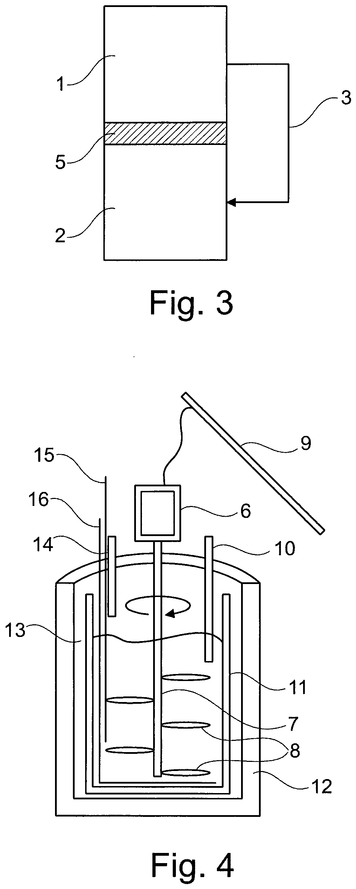

Eine bevorzugte Ausführungsform des mixotrophen Verfahrens zeigt Fig. 3, wobei die Algenölproduktion 1 und der artifizielle Pansen 2 durch eine luftundurchlässige Membran 5, wobei es sich bevorzugt um eine Lehm- oder Erdschicht handelt, voneinander getrennt sind, welche für die im artifiziellen Pansen 2 entstehenden Metabolite durchlässig ist. So wird eine Diffusion der Metabolite von der zweiten Stufe in die erste Stufe ermöglicht. Die bei der Algenölproduktion entstandene Biomasse wird über eine Auslass- bzw. Zufuhrvorrichtung 3 dem artifiziellen Pansen zugeführt

Fig. 4 zeigt einen Reaktor, welcher für die Verwendung als artifizieller Pansen geeignet ist. Der Reaktor verfügt über mindestens einen Mischer, welcher einen Antriebsmotor 6, eine Welle 7 und mindestens ein Rotorblatt 8 umfasst und in einer bevorzugten Ausführungsform durch eine Solaranlage 9 betrieben wird. Außerdem verfügt der Reaktor über mindestens eine Vorrichtung zur Zufuhr von Biomasse sowie mindestens eine Vorrichtung zur Entnahme der niedermolekularen Verbindungen, bevorzugt über eine Ernte- und Zufütterungsstelle 10. Bevorzugt ist der Reaktor mit einer doppelten Wand ausgestattet, wobei sich zwischen einer Innenwand 11 und einer Außenwand 12 eine Wärmeisolierung 13 befindet. Außerdem ist der Reaktor bevorzugt mit einem Zufuhrport für Reagenzien 14, Prozesssensorik 15 sowie einer Begasungsanlage 16 ausgestattet.

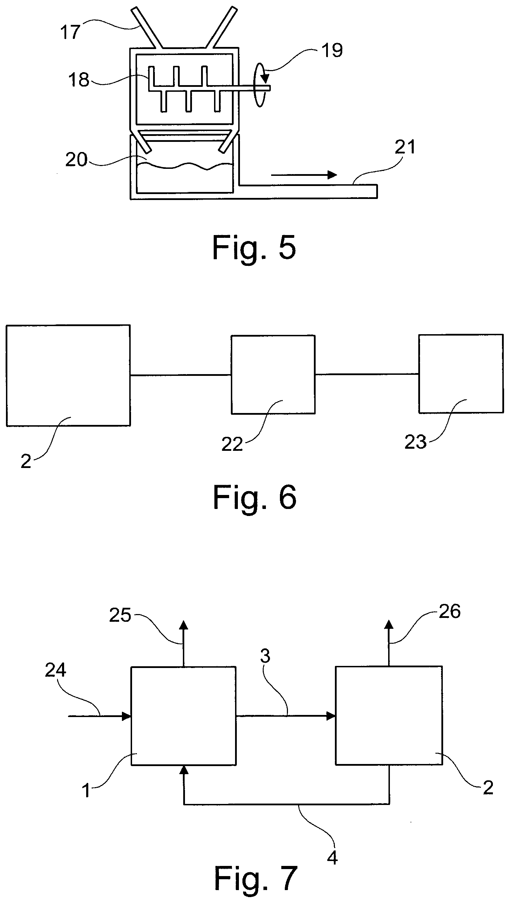

Bei Fig. 5 handelt es sich um eine Darstellung des in einer bevorzugten Ausführungsform dem artifiziellen Pansen vorgeschalteten Zerkleinerers, welcher einen Trichter 17, einen Häcksler 18, eine Antriebswelle des Häckslers 19, welche mittels Solaranlage, Strom oder Biogas betrieben werden kann, einen Auffangbehälter 20 sowie eine automatische Zufuhr 21 zum Zufütterungsport des artifiziellen Pansen umfasst.

Fig. 6 ist eine schematische Darstellung einer bevorzugten Ausführungsform eines dem artifiziellen Pansen 2 angeschlossenen Durchflusszytometers 23 mit zwischengeschalteter automatischer Probenentnahme 22, welche mit einer integrierten vollautomatischen Fluoreszenzfärbung ausgestattet ist. Das Durchflusszytometer ist mit einer vollautomatischen Detektion und Auswertung ausgestattet.

Fig. 7 zeigt eine schematische Darstellung möglicher Ausführungsformen der Erfindung. Über eine Zufuhrvorrichtung 24 werden Algen der aeroben ersten Stufe 1 (phototrophes Verfahren) zugeführt. Es ist mindestens eine Auslassvorrichtung 25 vorhanden, welche die Entnahme der in der ersten Stufe hergestellten Biokraftstoffe ermöglicht. Die in der ersten Stufe entstandene Biomasse wird über eine Auslassvorrichtung 3 der ersten Stufe entnommen und dem artifiziellen Pansen 2 über eine Zufuhrvorrichtung 3 zugeführt, wo sie in einem anaeroben Prozess zu niedermolekularen Verbindungen verstoffwechselt wird. Diese werden über eine Auslassvorrichtung 26 entnommen und gegebenenfalls direkt raffiniert. Über eine Auslass- bzw. Zufuhrvorrichtung 4, welche den artifiziellen Pansen 2 mit der Algenölproduktion 1 verbindet, wobei es sich bevorzugt um eine luftundurchlässige Membran handelt, bevorzugt um eine Lehm- oder Erdschicht, können in einer bevorzugten Ausführungsform im artifiziellen Pansen entstehende Metabolite in die erste Stufe gelangen.

## Patentansprüche

1. Verfahren zur Herstellung von Biokraftstoffen, umfassend die Schritte
a. in einer ersten Stufe (1) die Produktion von Biokraftstoffen aus Organismen ausgewählt aus Makroalgen, Mikroalgen, ethanolproduzierenden Algen und/oder Archaeen unter Bildung von Biomasse, und
b. in einer zweiten Stufe (2) die Umsetzung der Biomasse aus der ersten Stufe zu niedermolekularen Verbindungen in einem Reaktor, welcher im Pansen von Wiederkäuern vorkommende Mikroorganismen enthält,
**dadurch gekennzeichnet, dass** es sich bei der ersten Stufe um einen aeroben Prozess und bei der zweiten Stufe um einen anaeroben Prozess handelt und dass die erste aerobe Stufe von der zweiten anaeroben Stufe durch eine Vorrichtung getrennt ist,
welche eine Diffusion der in der zweiten Stufe entstehenden Metabolite in die erste Stufe ermöglicht.

2. Verfahren gemäß Anspruch 1, **dadurch gekennzeichnet, dass** in der ersten Stufe Biodiesel, Bioethanol, Biogas und/oder Biowasserstoff hergestellt werden.

3. Verfahren gemäß einem der vorhergehenden Ansprüche, **dadurch gekennzeichnet, dass** die in der zweiten Stufe hergestellten niedermolekularen Verbindungen Methan, Wasserstoff, Butanol, Ethanol, Essigsäure, Aceton, Buttersäure und/oder Milchsäure sind.

4. Verfahren gemäß einem der vorhergehenden Ansprüche, **dadurch gekennzeichnet, dass** die Organismen der ersten Stufe mindestens eine der Gattungen und/oder Arten ausgewählt aus der Gruppe umfassend Braunalgen, Laminaria ssp., Palmaria ssp., Sargassum ssp., Gracilaria ssp., Chlorella ssp., Dunaliella ssp., Prymnesium parvum, Euglena ssp. und Archaeen umfassen.

5. Verfahren gemäß einem der vorhergehenden Ansprüche, **dadurch gekennzeichnet, dass** die zweite Stufe mindestens einen der Mikroorganismen ausgewählt aus der Gruppe umfassend Ruminococcus ssp., Ralstonia ssp., Lactobacillus ssp., Clostridium ssp., Bacterioides ssp., Archaeen, Isotrichidae, Ophryoscolecidae, Ciliata, Hefen und Pilze, umfasst.

6. Verfahren gemäß einem der vorhergehenden Ansprüche, **dadurch gekennzeichnet, dass** die Mikroorganismen der zweiten Stufe mindestens eine Bakterienart umfasst, ausgewählt aus der Gruppe umfassend Clostridium acetobutylicum, Clostridium beijerinckii, Ruminococcus flavefaciens, Ruminococcus albus, Ralstonia eutropha und Hydrogenomonas eutrophus.

7. Verfahren gemäß einem der vorhergehenden Ansprüche, **dadurch gekennzeichnet, dass** die erste Stufe sowohl im geschlossenen System als auch im offenen System durchgeführt werden kann.

8. Verfahren gemäß einem der Ansprüche 1 bis 7, **dadurch gekennzeichnet, dass** der Reaktor, welcher im Pansen von Wiederkäuern vorkommende Mikroorganismen enthält, sich sowohl für einen Chargenprozess als auch für ein Zulauf-Verfahren eignet, wobei der Reaktor aufweist:
a. eine Vorrichtung (10; 3) zur Zufuhr von Biomasse und gegebenenfalls Reagenzien,
b. eine Vorrichtung (10; 26) zur Entnahme der niedermolekularen Verbindungen und
c. eine Begasungsanlage (16).

9. Verfahren gemäß Anspruch 8, **dadurch gekennzeichnet, dass** der Reaktor mit Prozesssensorik ausgestattet ist, welche mindestens eine der folgenden Komponenten umfasst:
Einen pH-Wert-, CO₂-, O₂-, H₂-Sensor, Photometer, Kontaktsensoren, Durchflusszytrometrie und/oder eine elektronische Nase,
wobei mindestens eine der Komponenten über einen Rückkopplungsmechanismus verfügt.

10. System zur Herstellung von Biokraftstoffen, aufweisend
a. einen Reaktor (1) zur Produktion von Biokraftstoffen aus Organismen ausgewählt aus Makroalgen, Mikroalgen, ethanolproduzierenden Algen und/oder Archaeen in einer ersten Stufe in einem aeroben Bereich unter Bildung von Biomasse, und
b. einen Reaktor (2) zur Umsetzung der Biomasse aus dem Reaktor (1) zu niedermolekularen Verbindungen in einer zweiten Stufe in einem anaeroben Bereich, welcher im Pansen von Wiederkäuern vorkommende Mikroorganismen enthält,
wobei
a. der Reaktor (1) einen Einlass (24) zum Zuführen der Organismen, einen Auslass (25) zur Abgabe der erzeugten Biokraftstoffe, einen Auslass (3) zur Abgabe der erzeugten Biomasse an den Reaktor (2) und einen Einlass (4) zur Rückführung der im Reaktor (2) erzeugten Metabolite in Form einer Membran aufweist und
b. der Reaktor (2) einen Einlass (3) zum Zuführen der Biomasse aus dem Reaktor (1), einen Auslass (4) zur Rückführung der erzeugten Metabolite in den Reaktor (1) in Form einer Membran und einen Auslass (26) für die erzeugten niedermolekularen Verbindungen aufweist.

## Claims

1. Process for producing biofuels comprising the steps of
a. in a first step (1), the production of biofuels from organisms selected from macroalgae, microalgae, ethanol-producing algae and/or archaea, with the formation of biomass, and
b. in a second step (2), the conversion of the biomass from the first step into low-molecular-weight compounds in a reactor containing microorganisms found in the rumen of ruminants,
**characterized in that** the first step is an aerobic process and the second step is an anaerobic process, and that the first aerobic step is separated from the second anaerobic step by a device that allows diffusion of the metabolites formed in the second step into the first step.

2. Process according to Claim 1, **characterized in that** biodiesel, bioethanol, biogas and/or biohydrogen are produced in the first step.

3. Process according to either of the preceding claims, **characterized in that** the low-molecular-weight compounds produced in the second step are methane, hydrogen, butanol, ethanol, acetic acid, acetone, butyric acid and/or lactic acid.

4. Process according to any of the preceding claims, **characterized in that** the organisms in the first step include at least one of the genera and/or species selected from the group comprising brown algae, Laminaria spp., Palmaria spp., Sargassum spp., Gracilaria spp., Chlorella spp., Dunaliella spp., Prymnesium parvum, Euglena spp. and archaea.

5. Process according to any of the preceding claims, **characterized in that** the second step includes at least one of the microorganisms selected from the group comprising Ruminococcus spp., Ralstonia spp., Lactobacillus spp., Clostridium spp., Bacteroides spp., archaea, Isotrichidae, Ophryoscolecidae, Ciliata, yeasts and fungi.

6. Process according to any of the preceding claims, **characterized in that** the microorganisms in the second step include at least one bacterial species selected from the group comprising Clostridium acetobutylicum, Clostridium beijerinckii, Ruminococcus flavefaciens, Ruminococcus albus, Ralstonia eutropha and Hydrogenomonas eutrophus.

7. Process according to any of the preceding claims, **characterized in that** the first step may be carried out both in a closed system and in an open system.

8. Process according to any of Claims 1 to 7, **characterized in that** the reactor containing microorganisms found in the rumen of ruminants is suitable both for a batch process and for a fed-batch process, wherein the reactor includes:
a. a device (10; 3) for supplying the biomass and optionally reagents,
b. a device (10; 26) for withdrawing the low-molecular-weight compounds and
c. a gas-supply unit (16).

9. Process according to Claim 8, **characterized in that** the reactor is fitted with process sensors that include at least one of the following components:
A pH sensor, CO₂ sensor, O₂ sensor, H₂ sensor, photometer, contact sensors, flow cytometry and/or an electronic nose,
wherein at least one of the components is equipped with a feedback mechanism.

10. System for producing biofuels comprising
a. a reactor (1) for producing biofuels from organisms selected from macroalgae, microalgae, ethanol-producing algae and/or archaea in a first step in an aerobic region, with the formation of biomass, and
b. a reactor (2) for converting the biomass from reactor (1) into low-molecular-weight compounds in a second step in an anaerobic region containing microorganisms found in the rumen of ruminants,
wherein
a. reactor (1) has an inlet (24) for supplying the organisms, an outlet (25) for discharging the biofuels generated, an outlet (3) for discharging the biomass generated into reactor (2) and an inlet (4) for returning the metabolites generated in reactor (2), in the form of a membrane, and
b. reactor (2) has an inlet (3) for supplying the biomass from reactor (1), an outlet (4) for returning the metabolites generated to reactor (1), in the form of a membrane, and an outlet (26) for the low-molecular-weight compounds generated.

## Revendications

1. Procédé pour la fabrication de biocarburants, comprenant les étapes suivantes :
a. dans une première étape (1), la production de biocarburants à partir d'organismes choisis parmi les macroalgues, les microalgues, les algues produisant de l'éthanol et/ou les archées avec formation de biomasse, et
b. dans une deuxième étape (2), la transformation de la biomasse de la première étape en composés de faible poids moléculaire dans un réacteur, qui contient des microorganismes présents dans la panses de ruminants, **caractérisé en ce que** la première étape consiste en un processus aérobie et la deuxième étape consiste en un processus anaérobie et **en ce que** la première étape aérobie est séparée de la deuxième étape anaérobie par un dispositif qui permet une diffusion des métabolites formés dans la deuxième étape dans la première étape.

2. Procédé selon la revendication 1, **caractérisé en ce que** du biodiesel, du bioéthanol, du biogaz et/ou du biohydrogène sont préparés dans la première étape.

3. Procédé selon l'une quelconque des revendications précédentes, **caractérisé en ce que** les composés de faible poids moléculaire préparés dans la deuxième étape sont du méthane, de l'hydrogène, du butanol, de l'éthanol, de l'acide acétique, de l'acétone, de l'acide butyrique et/ou de l'acide lactique.

4. Procédé selon l'une quelconque des revendications précédentes, **caractérisé en ce que** les organismes de la première étape comprennent au moins un des genres et/ou des espèces choisis dans le groupe comprenant les algues brunes, Laminaria ssp., Palmaria ssp., Sargassum ssp., Gracilaria ssp., Chlorella ssp., Dunaliella ssp., Prymnesium parvum, Euglena ssp. et les archées.

5. Procédé selon l'une quelconque des revendications précédentes, **caractérisé en ce que** la deuxième étape comprend au moins un des microorganismes choisis dans le groupe comprenant Ruminococcus ssp., Ralstonia ssp., Lactobacillus ssp., Clostridium ssp., Bacterioides ssp., les archées, les Isotrichidés, les Ophryoscolecidés, les Ciliés, les levures et les champignons.

6. Procédé selon l'une quelconque des revendications précédentes, **caractérisé en ce que** les microorganismes de la deuxième étape comprennent au moins une espèce bactérienne choisie dans le groupe comprenant Clostridium acetobutylicum, Clostridium beijerinckii, Ruminococcus flavefaciens, Ruminococcus albus, Ralstonia eutropha et Hydrogenomonas eutrophus.

7. Procédé selon l'une quelconque des revendications précédentes, **caractérisé en ce que** la première étape peut être réalisée aussi bien dans le système fermé que dans le système ouvert.

8. Procédé selon l'une quelconque des revendications 1 à 7, **caractérisé en ce que** le réacteur, qui contient des microorganismes présents dans la panse de ruminants, est approprié aussi bien pour un processus par lots que pour un procédé à alimentation, le réacteur comprenant :
a. un dispositif (10 ; 3) pour l'introduction de biomasse et éventuellement de réactifs,
b. un dispositif (10 ; 26) pour le soutirage des composés de faible poids moléculaire et
c. une unité de gazage (16).

9. Procédé selon la revendication 8, **caractérisé en ce que** le réacteur est équipé de capteurs de processus, qui comprennent au moins un des composants suivants :
un capteur de pH, de CO₂, d'O₂, d'H₂, un photomètre, des capteurs de contact, une cytométrie en flux et/ou un nez électronique,
au moins un des composants disposant d'un mécanisme de rétroaction.

10. Système pour la fabrication de biocarburants, comprenant :
a. un réacteur (1) pour la production de biocarburants à partir d'organismes choisis parmi les macroalgues, les microalgues, les algues produisant de l'éthanol et/ou les archées dans une première étape dans une zone aérobie avec formation de biomasse, et
b. un réacteur (2) pour la transformation de la biomasse issue du réacteur (1) en composés de faible poids moléculaire dans une deuxième étape dans une zone anaérobie, qui contient des microorganismes présents dans la panse de ruminants,
dans lequel
a. le réacteur (1) comprenant une entrée (24) pour l'introduction des organismes, une sortie (25) pour le déchargement des biocarburants formés, une sortie (3) pour le déchargement de la biomasse formée vers le réacteur (2) et une sortie (4) pour le recyclage des métabolites formés dans le réacteur (2) sous la forme d'une membrane, et
b. le réacteur (2) comprenant une entrée (3) pour l'introduction de la biomasse issue du réacteur (1), une sortie (4) pour le recyclage des métabolites formés dans le réacteur (1) sous la forme d'une membrane et une sortie (26) pour les composés de faible poids moléculaire formés.
